# FASCICULE DE BREVET EUROPEEN

(11) **EP 0 706 995 B1**
(45) Date de publication et mention de la délivrance du brevet: **27.12.1996**
(21) Numéro de dépôt: 95402035.0
(22) Date de dépôt: 08.09.1995
(51) Int. Cl.: C07C 235/60, C07C 233/85, C07C 233/49, A61K 7/48

(54) **Utilisation de dérivés de déhydroalanines pour protéger la peau, les muqueuses et/ou les cheveux du stress oxydant, compositions cosmétiques ou dermatologiques les contenant et composés nouveaux utilisés**
Verwendung von Dehydroalanin-Derivate zum Schutz der Haut, der Schleimhaut und/oder des Haares gegen oxidativen Stress, diese enthaltende kosmetische oder dermatologische Zusammensetzungen und ähnliche neue Verbindungen
Use of dehydroalanine-derivatives for the protection of the skin, mucosa and/or hair against oxidative stress, cosmetic or dermatologic compositions containing them and new compounds useful therein

(30) Priorité: 15.09.1994 FR 9411029
(43) Date de publication de la demande: 17.04.1996
(73) Titulaire: L'OREAL, 75008 Paris (FR)
(72) Inventeur: Genard, Sylvie, F-75012 Paris (FR); Galey, Jean-Baptiste, F-93600 Aulnay-sous-Bois (FR); Hocquaux, Michel, F-75012 Paris (FR)
(74) Mandataire: Casalonga, Axel

(56) Documents cités:
- GB-A- 1 354 571
- FREE RAD. RES. COMMS., vol. 5, no. 3, 1988 pages 159-168, P. BUC-CALDERON ET AL 'INHIBITION OF O2.-AND HO.- MEDIATED PROCESSES BY A NEW CLASS OF FREE RADICAL SCAVENGERS: THE N-ACYL DEHYDROALANINES'
- JOURNAL OF POLYMER SCIENCE: PART C: POLYMER LETTERS, vol. 24, 1986 pages 199-206, K.A. BRANDT ET AL 'SYNTHESIS OF BIS AND TRIS (DEHYDROALANINE) CROSSLINKING AGENTS AND THEIR USE IN THE PREPARATION OF HYDROPHILIC NUCLEIC ACID BASE RESINS'

## Description

La présente invention concerne des dérivés de déhydroalanines, utiles pour protéger la peau, les muqueuses et/ou les cheveux contre le stress oxydant, ainsi que les compositions cosmétiques ou dermatologiques contenant de tels composés.

Dans le domaine de la santé et de la cosmétique, le concept du stress oxydant est connu, stress oxydant qui apparaît notamment dès qu'il existe un déséquilibre de la balance antioxydant-prooxydant. Ce déséquilibre se traduit notamment par des processus oxydatifs non contrôlés au sein des tissus vivants; ces processus mettent en jeu des radicaux libres oxygénés et conduisent notamment à des dégats oxydatifs sur les molécules et les macromolécules biologiques (Sies, H., In Oxidative Stress, Academic Press Inc. (London) Ltd, 1985).

Il est connu que des situations diverses provoquent, favorisent ou accompagnent le stress oxydant ou en sont la conséquence; il s'agit notamment de l'exposition aux rayons ultraviolets et aux rayonnements ionisants, du vieillissement, de la carcinogénèse, de la toxicité et/ou du mode d'action de certains médicaments.

Les radicaux libres oxygénés sont des espèces très instables et très réactives. Ainsi, dans les milieux biologiques, ils réagissent avec tous types de molécules ou macromolécules avoisinantes (sucres, protéïnes, lipides, nucléotides...) causant les dégâts oxydatifs précédemment cités.

Afin de protéger les tissus biologiques de ces dégradations irréversibles, plusieurs stratégies peuvent être envisagées. On peut par exemple agir sur l'une des étapes de formation des radicaux libres oxygénés. On peut également envisager la protection des tissus biologiques en stabilisant ces espèces activées de l'oxygène. Ce concept est par exemple illustré par la théorie des molécules dites capto-datives.

Selon cette théorie, puisque les carbocations sont stabilisés par des substituants électrodonneurs et les carbanions sont stabilisés par des substituants électroaccepteurs, on peut imaginer la stabilisation de centres radicalaires substitués à la fois par un substituant électroaccepteur et par un substituant électrodonneur. Les molécules ainsi substituées sont dites capto-datives.

Cette théorie a été par exemple vérifiée pour les molécules capto-datives de formule (II) mises en présence du radical isobutyronitrile (L. STELLA, Z.JANOUSEK, R.MERENYI, H.G. VIEHE, Angew. Chem. Int. Ed. Engl., 1978, 17, 691) 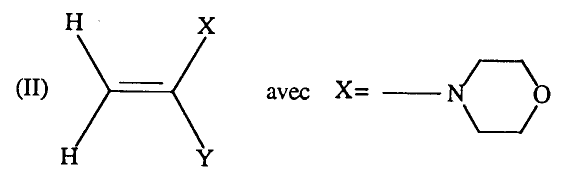 et Y= ― CN
ou X= ― SMe et Y= ― CN D'autres oléfines de type (II) ont été très étudiées pour leurs propriétés radicophiles vis-à-vis des radicaux oxygénés.

Ainsi, les N-acyldéhydroalanines, molécules capto-datives de formule (III): 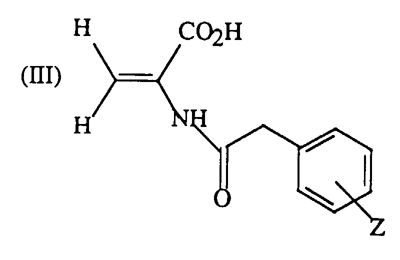 où Z=H, -OMe par exemple ont été étudiées pour leurs propriétés radicophiles vis-à-vis des radicaux oxygénés. On peut se référer à ce sujet à l'article de P. BUCCALDERON et M.ROBERFOID, Free Rad. Res. Commun., 1988, 5, 159-68.

Ces molécules (III) possèdent en plus du site capto-datif deux sites susceptibles de réagir avec les radicaux oxygénés: un site proradicalaire (réactivité par abstraction d'hydrogène) et un noyau aromatique (addition de OH°) :

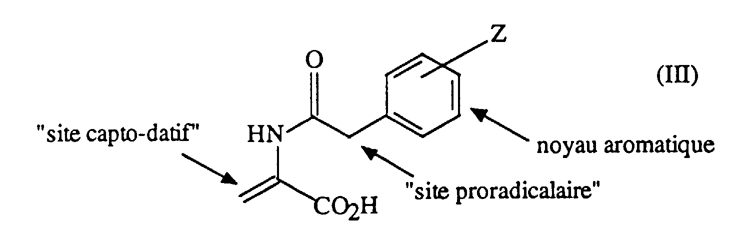

Les composés de formule (III) sont utilisés par exemple pour la prévention et le traitement des cancers, comme décrit dans le document EP-A-1 13330.

De façon surprenante, il a été maintenant découvert que les composés définis par la formule (I) ci-dessous : 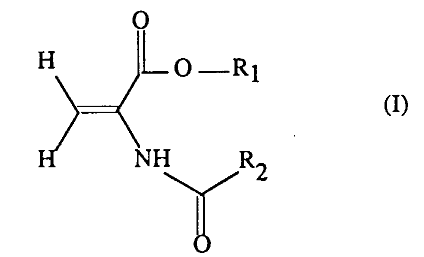 avec
R₁ : H, ou alkyle en C₁ à C₄ à chaîne linéaire ou ramifiée, 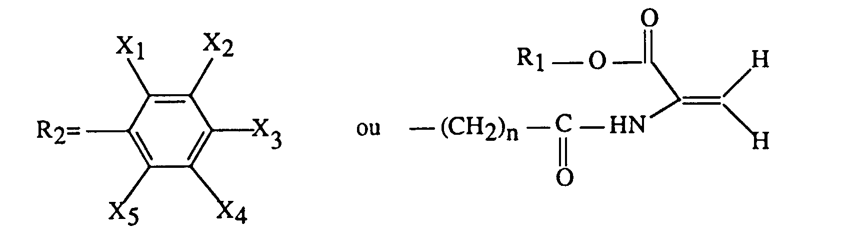 ou alkyle en C₁-C₂₀ à chaîne linéaire ou ramifiée où X₁,X₂,X₃,X₄,X₅ = H, halogène, hydroxyle, alkyle ou alcoxy en C₁-C₄ à chaîne linéaire ou ramifiée, -OSO₃H, 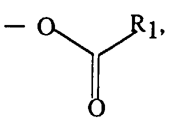 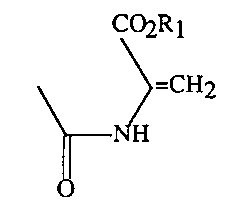 et n = 2, 3 ou 4,
ainsi que leurs sels (lorsque R₁ = H) de cations non toxiques incluant les cations alcalins et alcalino-terreux tels que sodium, lithium, potassium, calcium, magnésium et les sels d'ammonium,
avaient la propriété de protéger les milieux biologiques en situation de stress oxydant aussi efficacement que les déhydroalanines de formule (III), et même plus efficacement à basse concentration. Ceci est d'autant plus surprenant que l'on pouvait penser que la présence du groupe méthylène dans les déhydroalanines de formule (III) était essentielle en ce sens qu'elle crée, en plus de celui constitué par la double liaison, un site supplémentaire permettant de capter des radicaux libres, comme l'indique l'article de Buc-Caldéron indiqué ci-dessus.

Dans les composés de formule (I) l'halogène est de préférence du chlore, mais peut être aussi du brome ou du fluor. Les radicaux alkyle ou alcoxy en C₁-C₄ sont de préférence méthyle, éthyle, méthoxy ou éthoxy. Les radicaux alkyle en C₁-C₂₀ sont de préférence hexyle, heptyle, octyle, décyle, tétradécyle et octadécyle.

Les composés de formule (I) ont une excellente activité protectrice vis-à-vis de cellules en culture soumises à un stress oxydant, à des concentrations relativement faibles. Certains de ces composés sont actifs à une concentration de 0,1 µM, concentration à laquelle ils ont une meilleure activité protectrice qu'une N-acyldéhydroalanine de formule (III) dans laquelle Z=-OCH₃ en position para.

On peut donc avantageusement utiliser les composés de formule (I) à faible concentration, ce qui présente l'avantage de minimiser ainsi les effets secondaires possibles de ces composés car l'on sait que l'activité d'un composé est toujours liée à une certaine toxicité.

La présente invention a ainsi pour objet l'utilisation des dérivés de déhydroalanines de formule (I) ci-dessus pour protéger la peau, les cheveux et/ou les muqueuses contre le stress oxydant.

Un autre objet est constitué par les compositions cosmétiques ou dermatologiques mettant en oeuvre ces composés.

Parmi les déhydroalanines de formule (I), certains composés sont nouveaux. Il s'agit des composés de formule générale : 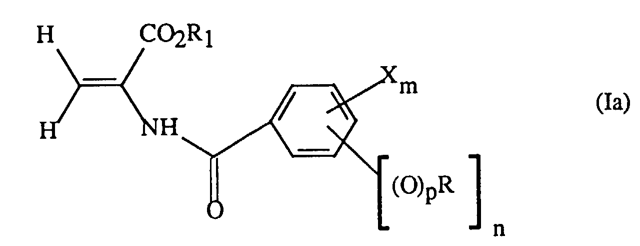 dans laquelle R₁ désigne l'hydrogène,
X désigne un halogène,
R désigne un radical alkyle en C₁-C₄ à chaîne linéaire ou ramifiée,
n + m = 1 à 5, m pouvant être nul,
p = 0 ou 1, et de leurs sels de métaux alcalins ou alcalino-terreux ou d'ammonium.

A titre de radicaux R, les radicaux méthyle et éthyle sont préférés.

L'atome d'halogène X est de préférence du chlore, mais peut également désigner du brome ou du fluor.

Parmi les composés nouveaux de formule (I), on peut également citer les composés de formule (Ib) suivante : 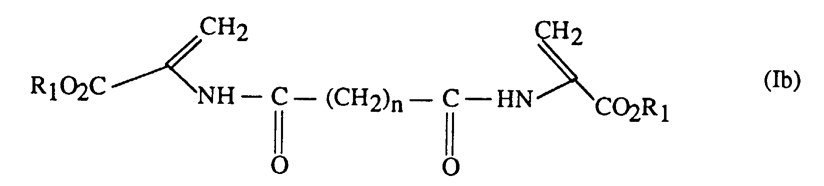 avec n = 2, 3 ou 4 et R₁ = H, ou alkyle en C₁-C₄ à chaîne linéaire ou ramifiée, notamment la déhydroalanine dérivée du succinamide ayant la formule suivante : 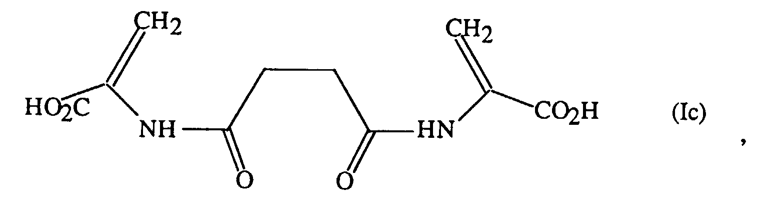 ainsi que leurs sels de métaux alcalins ou alcalino-terreux ou d'ammonium.

L'invention a donc également pour objet les composés nouveaux de formules (Ia) et (Ib) ci-dessus.

Les déhydroalanines de formules (I), (Ia) et (Ib) sont préparées selon un procédé choisi parmi les procédés mentionnés dans la littérature pour la préparation de ce type de composés. On peut les préparer par exemple, en condensant un amide approprié avec l'acide pyruvique ou son ester, au reflux d'un solvant organique convenable, comme décrit dans les documents Bull. Acad. Sci. USSR, 1955, p. 231 ou Chem. Ber. 1957, volume 90, p. 194; ou bien en passant par une chloramine intermédiaire dérivée de l'alanine, comme décrit par exemple dans le document Synthesis, 1977, p. 457; ou encore en passant par une chloramine intermédiaire dérivée de l'acide aspartique ou de son ester, comme décrit par exemple dans le document Agric. Biol. Chem., 1984, volume 48, p. 1251-5; ou encore par déshydratation de dérivés de la sérine, soit par le carbonyldiimidazole, comme décrit par exemple dans Synthesis, 1982, p. 968, soit par des carbodiimides divers comme décrit par exemple dans le document J. Org. Chem., 1980, volume 45, p. 3131-2, soit par formation d'un groupe scindable mésylate, comme décrit par exemple dans le document Bull. Chem. Soc. Jpn., 1981, volume 54, p. 1132 ou tosylate comme décrit par exemple dans J. Am. Chem. Soc., 1963, volume 85, p. 1123; ou enfin par déshydrohalogénation de (β)-chloroalanines, comme décrit par exemple dans le document J. Polym. Sci., Part C: Polym. Lett., 1986, 24, 199.

D'autres objets de l'invention apparaîtront à la lecture de la description et des exemples qui suivent.

Les exemples suivants illustrent les procédés de préparation des composés nouveaux de formules (Ia) et (Ib).

### EXEMPLES

### EXEMPLE 1

### Préparation de la N-ortho-toluyl déhydroalanine (synthèse en 2 étapes)

### 1ère étape :

Dans un ballon tricol de 500 ml équipé d'un pH-mètre et de deux ampoules d'introduction contenant d'une part une solution aqueuse de soude 2,5N et d'autre part le chlorure d'o-toluyle, on dissout 20 g d'acide D,L-aspartique (ALDRICH) dans 210 ml d'un mélange solution aqueuse de soude 2,5N (120 ml) - THF (90 ml). On additionne goutte à goutte le chlorure d'o-toluyle en maintenant le pH supérieur à 9 par addition simultanée de la solution de soude 2,5N. On maintient sous agitation pendant 2h 30 minutes après la fin de l'addition du chlorure d'acide avant d'acidifier le milieu réactionnel par une solution d'acide chlorhydrique concentré. Le milieu est épuisé par l'acétate d'éthyle. La phase organique est lavée, séchée et concentrée à sec pour fournir 27,9 g. (Rendement non optimisé 74%) d'acide N-ortho-toluyl-aspartique utilisé tel quel pour la seconde étape.

**RMN** ^{**1**}**H 400MHz** (DMSOd6, δ ppm) : 2,34 (s, 3H, CH₃), 2,67 (d.d, 1H, H2a), 2,82 (d.d, 1H, H2b), 4,72 (m, 1H, H3), 7,23 (m, 2H, H8 et H10), 7,31 (m, 2H, H9 et H11), 8,48 (d, 1H, H4), 12,53 (s, 2H, OH).

**RMN** ^{**13**}**C 100MHz** (DMSOd6, δ ppm) : 19,18 (CH3), 35,75 (C2), 49,01 (C3), 125,35 (C10), 126,99 (C11), 129,34 (C8), 130,33 (C9), 135,30 (C6), 136,58 (C7), 168,83 (C5), 171,68 (C1 ou C12), 172,39 (C1 ou C12).

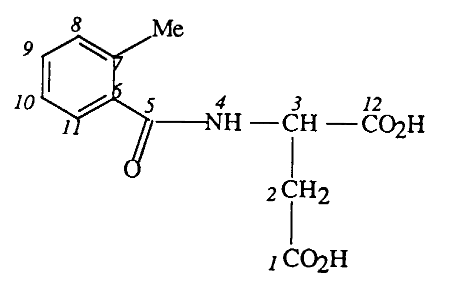

| **Analyse élémentaire :** | | | | |
|---|---|---|---|---|
| | **C**% | **H**% | **N**% | **O**% |
| *Calculé* | 57,37 | 5,22 | 5,58 | 31,84 |
| *Trouvé* | 57,21 | 5,01 | 5,37 | 31,82 |

**Point de fusion** : 160°C (Kofler)

### 2ème étape :

Dans un ballon tricol de 500 ml équipé d'un thermomètre, d'une ampoule d'introduction et d'un réfrigérant, on dissout 10 g d'acide N-ortho-toluyl-aspartique dans 20 ml de méthanol. Après complète dissolution, 28,5 ml d'une solution aqueuse d'hypochlorite de sodium titrant 1,40 mol/l sont additionnés goutte à goutte en maintenant la température inférieure à 20°C. A la fin de l'addition, le milieu est dilué par 180 ml de méthanol avant d'être porté au reflux pendant 1h 30 minutes. Après concentration sous vide, le résidu est repris dans l'acétate d'éthyle et extrait par une solution aqueuse de bicarbonate de sodium. La phase aqueuse est acidifiée par l'acide chlorhydrique et extraite par l'acétate d'éthyle. La phase organique est lavée par la saumure avant l'être séchée et concentrée sous vide pour fournir une huile qui cristallise par addition d'eau. Le précipité formé est filtré, purifié par recristallisation dans l'eau (additionnée de quelques gouttes de méthanol si nécessaire) pour fournir 2,65 g de N-ortho-toluyl déhydroalanine. (Rendement non optimisé 32%).

**RMN** ^{**1**}**H 500MHz** (DMSOd6, δ ppm) : 2,38 (s, 3H, H12), 5,77 (s, 1H, H11b), 6,13 (s, 1H, H11a), 7,26 à 7,32 (m, 2H, H3 et H5), 7,37 à 7,40 (t.d, 1H, H4), 7,40 à 7,45 (d.d, 1H, H6), 9,23 (s, 1H, NH), 13,28 (s, 1H, H10).

**RMN** ^{**13**}**C 125MHz** (DMSOd6, δ ppm): 19,42 (C12), 109,81 (C11), 125,68 (C5), 127,12 (C6), 130,00 (C4), 130,71 (C3), 133,93 (C9), 135,64 (C2), 135,91 (C1), 165,03 (C10), 167,90 (C7).

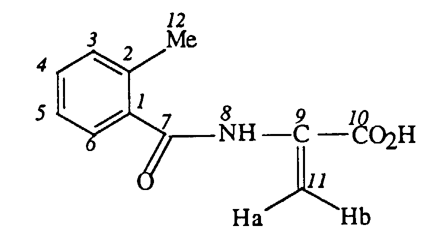

| **Analyse élémentaire :** | | | | |
|---|---|---|---|---|
| | **C**% | **H**% | **N**% | **O**% |
| *Calculé* | 64,38 | 5,40 | 6,83 | 23,39 |
| *Trouvé* | 64,33 | 5,45/5,36 | 6,80 | 23,27 |

**Point de fusion** : 108°C (Kofler)

### EXEMPLE 2

### Préparation de la N-(2-méthoxy-5-chlorobenzoyl)déhydroalanine (synthèse en 2 étapes)

### 1ère étape :

Dans un ballon tricol de 500 ml équipé d'un pH-mètre et de deux ampoules d'introduction contenant d'une part une solution aqueuse de soude 2,5N et d'autre part le chlorure de 2-méthoxybenzoyle, on dissout 10 g d'acide D,L-aspartique (ALDRICH) dans 105 ml d'un mélange solution aqueuse de soude 2,5N (60 ml) - THF (45 ml). On additionne goutte à goutte le chlorure de 2-méthoxybenzoyle en maintenant le pH supérieur à 9 par addition simultanée de la solution de soude 2,5N. On maintient sous agitation pendant 3h après la fin de l'addition du chlorure d'acide avant d'acidifier le milieu réactionnel par une solution d'acide chlorhydrique concentré. Le milieu est épuisé par l'acétate d'éthyle. Le phase organique est lavée, séchée et concentrée à sec pour fournir l'acide N-(2-méthoxybenzoyl)aspartique brut. Le produit peut être utilisé tel quel pour la seconde étape. (Rendement non optimisé 75%) ou purifié par lavage par un solvant organique convenable.

**RMN** ^{**1**}**H 500MHz** (DMSOd6, δ ppm) : 2,83 (m, 2H, H9), 3,92 (s, 3H, H12); 4,80 (m, 1H, H8), 7,07 (d.d.d, 1H, H4), 7,18 (d.d, 1H, H2), 7,52 (d.d.d, 1H, H3), 7,91 (d.d, 1H, H5), 8,85 (d, 1H, NH), 12,68 (s, 2H, OH). 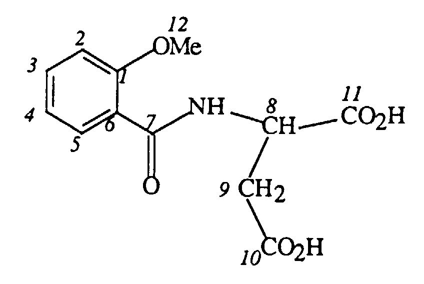 **Masse :** MH⁺ = 268

| **Analyse élémentaire :** | | | | |
|---|---|---|---|---|
| | **C**% | **H**% | **N**% | **O**% |
| *Calculé* | 53,95 | 4,90 | 5,24 | 35,92 |
| *Trouvé* | 53,98 | 4,95 | 5,15 | 35,80 |

**Point de fusion :** 196°C (Kofler)

### 2ème étape :

Dans un réacteur équipé d'un thermomètre, d'une ampoule d'introduction et d'un réfrigérant, on dissout l'acide N-(2-méthoxybenzoyl)aspartique dans du méthanol (2,7 ml/g). Après complète dissolution, on additionne goutte à goutte une solution aqueuse d'hypochlorite de sodium titrée en maintenant la température inférieure à 20°C. A la fin de l'addition, le milieu est dilué 2 fois par le méthanol avant d'être porté au reflux pendant 1h 30 minutes. Après concentration sous vide, le résidu est repris dans l'acétate d'éthyle et extrait par une solution aqueuse de bicarbonate de sodium. La phase aqueuse est acidifiée par l'acide chlorhydrique et extraite par l'acétate d'éthyle. La phase organique est lavée par la saumure avant d'être séchée et concentrée sous vide. Le résidu est solubilisé au reflux d'un mélange éthanol-eau 1:1. La N-(2-méthoxy-5-chlorobenzoyl) déhydroalanine cristallise au refroidissement. (Rendement non optimisé 4%).

**RMN** ^{**1**}**H 500MHz** (DMSOd6, δ ppm) : 4,02 (s, 3H, H12), 5,82-6,58 (s, 2H, H11), 7,30 (d, 1H, H6), 7,63 (d.d, 1H, H5), 7,94 (d, 1H, H3), 10,48 (s, 1H, NH), 13,68 (s, 1H, H10). 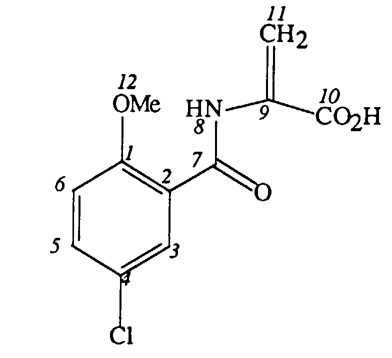 **Masse :** M⁺ = 255

| **Analyse élémentaire :** | | | | | |
|---|---|---|---|---|---|
| | **C**% | **H**% | **N**% | **O**% | **Cl**% |
| *Calculé* | 51,68 | 3,94 | 5,48 | 25,03 | 13,87 |
| *Trouvé* | 52,38 | 4,05 | 5,56 | 25,24 | |

**Point de fusion :** 210-214°C (Kofler)

### EXEMPLE 3

### Préparation de la N-(2-méthoxybenzoyl)déhydroalanine (synthèse en 1 étape)

Une suspension de 2-méthoxybenzamide dans le dichloro-1,2-éthane (24,5 ml/g) est portée au reflux dans un ballon surmonté d'un système permettant l'élimination de l'eau formée au cours de la réaction en présence de 3 équivalents d'acide pyruvique fraîchement distillé. Lorsque la formation d'eau est terminée (environ 7 heures), le milieu réactionnel est abandonné à température ambiante. Après filtration des insolubles, le filtrat est extrait par une solution aqueuse de bicarbonate de sodium. Les phases aqueuses réunies sont extraites par le dichlorométhane puis acidifiées par l'acide chlorhydrique. Le précipité formé est filtré, séché puis repris dans l'acétate d'éthyle. Après filtration de l'insoluble, le filtrat est concentré sous vide, solubilisé par le dichlorométhane puis précipité par de l'heptane fournissant ainsi la N-(2-méthoxybenzoyl)déhydroalanine pure. (Rendement non optimisé 8,5%).

**RMN** ^{**1**}**H 500MHz** (DMSOd6, δ ppm) : 4,01 (s, 3H, H12), 5,79 (s, 1H, H11a), 6,59 (s, 1H, H11b), 7,12 à 7,15 (d.d, 1H, H4), 7,25 à 7,26 (d.d, 1H, H6), 7,57 à 7,59 (d.d, 1H, H5), 8,01 à 8,03 (d.d, 1H, H3), 10,54 (s, 1H, NH), 13,66 (s, 1H, OH).

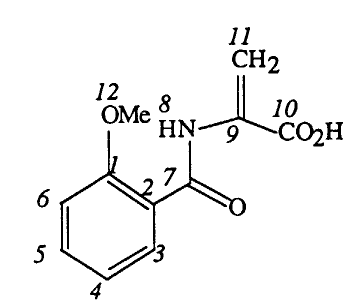

| **Analyse élémentaire :** | | | | |
|---|---|---|---|---|
| | **C**% | **H**% | **N**% | **O**% |
| *Calculé* | 59,73 | 5,01 | 6,33 | 28,93 |
| *Trouvé* | 59,72 | 5,07 | 6,46 | 28,85 |

**Point de fusion** : 132°C (Kofler)

### EXEMPLE 4

### Déhydroalanine dérivée du succinamide (Composé Ic)

Une suspension de succinamide dans le toluène (30 ml/g) est portée au reflux en présence de 6 équivalents d'acide pyruvique fraîchement distillé dans un ballon surmonté d'un appareil de Dean et Stark et d'un réfrigérant. Lorsque la formation d'eau est terminée (environ 5 heures), le milieu réactionnel est extrait par une solution aqueuse de bicarbonate de sodium. La phase aqueuse est acidifiée par l'acide chlorhydrique concentré pour précipiter la déhydroalanine. Après filtration, la déhydroalanine solide est lavée abondamment par un mélange éthanol/acétone puis 2 fois par l'acétate de méthyle avant d'être séchée sous vide au dessicateur ( Rendement non optimisé 2,3%).

**RMN** ^{**1**}**H 500MHz** (DMSOd6, δ ppm) : 2,59 (s, 4H, H5 & 5'), 5,66 (s, 2H, H2a & H2'a), 6,24 (s, 2H, H2b & H2'b), 9,09 (s, 2H, H3 & H3'), 13,26 (s, 2H, H1 & H1').

**RMN**^{**13**}**C 100MHz** (DMSOd6, δ ppm) : 30,94 (C₅, C_{5'}), 107,51 (C₂, C_{2'}), 133,06 (C₆, C₆,), 164,86 (C₁ et C₁, ou et et C₄,), 171,16 (C₁ et C_{1'} ou C₄ et C_{4'}).

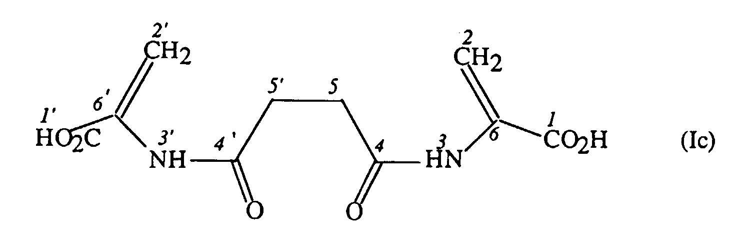

| **Analyse élémentaire :** | | | | |
|---|---|---|---|---|
| | **C**% | **H**% | **N**% | **O**% |
| *Calculé* | 46,88 | 4,72 | 10,93 | 37,47 |
| *Trouvé* | 46,37/46,46 | 4,90/5,0 | 10,57/10,54 | 36,50/36,36 |

**Point de fusion :** supérieur à 250°C (Kofler)

Les composés de formules (I), (Ia) et (Ib) et leurs sels peuvent être utilisés dans des compositions cosmétiques ou dermatologiques pour protéger la peau du stress oxydant.

Les compositions cosmétiques ou dermatologiques selon l'invention sont celles classiquement utilisées dans les domaines cosmétique ou dermatologique et peuvent être par exemple sous forme d'émulsions eau-dans-huile ou huile-dans-eau, de gels aqueux, alcooliques ou huileux ou de solutions aqueuses, hydroalcooliques ou alcooliques, en dispersion vésiculaire ou sous forme de mousses ou de sprays.

Les compositions cosmétiques ou dermatologiques selon l'invention mettent en oeuvre les composés de formules (I), (Ia) et (Ib) ou leurs sels, dans un milieu cosmétiquement ou dermatologiquement acceptable.

Ces compositions contiennent les composés de formules (I), (Ia) et (Ib) dans des proportions allant de 0,001 à 10% en poids, et de préférence de 0,01 à 2% en poids.

Le milieu cosmétiquement ou dermatologiquement acceptable est un milieu usuel dans le domaine cosmétique ou dermatologique.

Les compositions cosmétiques ou dermatologiques contenant les composés de formules (I), (Ia) ou (Ib) peuvent constituer notamment des crèmes de nettoyage, de protection, de traitement ou de soin pour le visage, pour les mains, ou pour le corps (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour soins capillaires, et notamment des shampooings, des crèmes ou des gels coiffants, des lotions ou des gels antichute, etc.

De façon connue, les compositions de l'invention peuvent contenir, outre des actifs hydrophiles ou lipophiles, des adjuvants habituels dans le domaine cosmétique ou dermatologique, tels que les gélifiants hydrophiles ou lipophiles, les conservateurs, les solvants, les parfums, les charges et les matières colorantes. Les quantités de ces différents adjuvants sont celles classiquement utilisées dans le domaine cosmétique ou dermatologique.

Les compositions cosmétiques ou dermatologiques selon l'invention peuvent contenir, en plus des composés de formules (I), (Ia) et (Ib), au moins un principe actif cosmétique ou dermatologique choisi plus particulièrement parmi les anti-inflammatoires, les antiacnéiques, les antipsoriasis, les antifongiques, les antibactériens, les anti-chute, les antipelliculaires, les filtres, les vitamines, les kératolytiques, les anti-oxydants, les hydratants, les anti-radicaux libres autres que les composés (I), (Ia) et (Ib), etc.

Les exemples suivants illustrent les compositions cosmétiques et dermatologiques selon l'invention.

### EXEMPLES

### Exemple 1

On prépare la composition suivante :
- Propylèneglycol 45,00 g
- N-benzoyldéhydroalanine 1,00 g
- KLUCEL H (hydroxypropylcellulose) 1,50 g
- Ethanol anhydre qsp 100 g

Cette composition se présente sous forme de gel destiné à être appliqué topiquement sur la zone de la peau à protéger.

### Exemple 2

On prépare la composition suivante :
- Monostéarate de glycérol 0,80 g
- Alcool cétylique 2,00 g
- Alcool cétylstéarylique 5,00 g
- Stéarate de polyoxyéthylène (vendu sous la dénomination de "MYRJ49") 3,00 g
- Acide acrylique réticulé par un agent poly-fonctionnel (vendu sous la dénomination de "CARBOPOL941") 0,50 g
- Triéthanolamine 0,30 g
- MIGLYOL 812 12,00 g
- Conservateur qs
- N-benzoyldéhydroalanine 0,5 g
- Eau qsp 100 g

Cette composition se présente sous forme de crème destinée à être appliquée topiquement sur la zone de la peau à protéger.

## Revendications

1. Utilisation d'une déhydroalanine de formule (I) 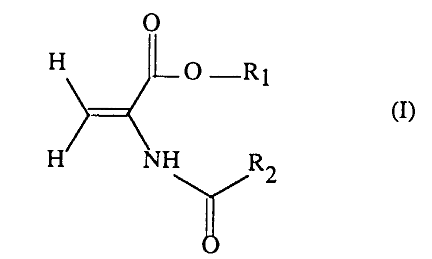 dans laquelle le radical R₁ désigne l'hydrogène, ou alkyle en C₁-C₄ à chaîne linéaire ou ramifiée, 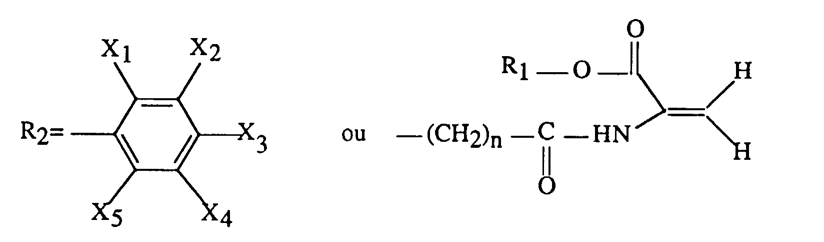 ou alkyle en C₁-C₂₀ à chaîne linéaire ou ramifiée où X₁,X₂,X₃,X₄,X₅ = H, halogène, hydroxyle, alkyle ou alcoxy en C₁-C₄ à chaîne linéaire ou ramifiée, -OSO₃H, 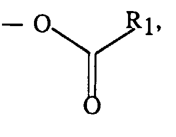 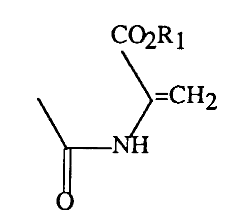 et n=2, 3 ou 4,
ou de ses sels de métaux alcalins ou alcalino-terreux ou d'ammonium, pour le traitement cosmétique de la peau, des muqueuses et/ou des cheveux en vue de les protéger contre le stress oxydant.

2. Utilisation selon la revendication 1 de composés de formule (I) dans laquelle l'halogène est du chlore.

3. Utilisation selon la revendication 1 de composés de formule (I) dans laquelle le radical alkyle ou alcoxy en C₁-C₄ est choisi parmi les radicaux méthyle, éthyle, méthoxy et éthoxy.

4. Composition cosmétique ou dermatologique, caractérisée par le fait qu'elle contient au moins un composé de formule (I) tel que défini dans l'une quelconque des revendications 1 à 3, dans un milieu cosmétiquement ou dermatologiquement acceptable.

5. Composition selon la revendication 4, caractérisée par le fait qu'elle contient 0,001% à 10% en poids, et de préférence 0,01 à 2% en poids d'au moins un composé de formule (I) tel que défini dans la revendication 1.

6. Composition selon la revendication 4 ou 5, caractérisée par le fait qu'elle comprend au moins un composé de formule (I) choisi parmi la N-benzoyldéhydroalanine, la N-ortho-toluyl déhydroalanine, la N-(2-méthoxy-5-chlorobenzoyl)déhydroalanine, la N-(2-méthoxybenzoyl)déhydroalanine et la déhydroalanine dérivée de succinamide répondant à la formule :

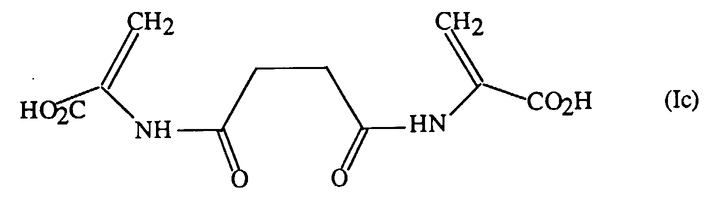

7. Composition cosmétique ou dermatologique selon l'une quelconque des revendications 4 à 6, caractérisée par le fait qu'elle contient en outre au moins un principe actif cosmétique ou dermatologique, choisi par exemple parmi les anti-inflammatoires, les anti-acnéiques, les anti-psoriasis, les anti-fongiques, les antibactériens, les anti-chute, les antipelliculaires, les filtres, les vitamines, les agents kératolytiques, les hydratants, les agents anti-radicaux libres autres que ceux de formule (I) et les antioxydants.

8. Dérivé de déhydroalanine de formule : 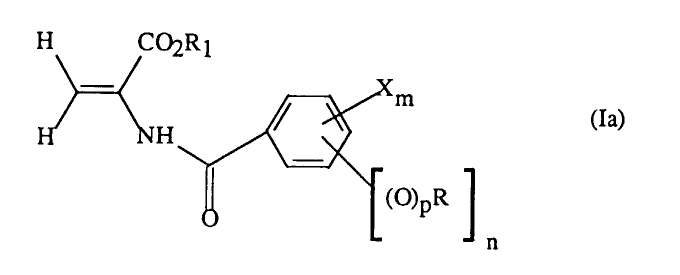 dans laquelle R₁ désigne l'hydrogène,
X désigne un halogène,
R désigne un reste alkyle en C₁-C₄ à chaîne linéaire ou ramifiée,
n + m = 1 à 5, m pouvant être nul,
p = 0 ou 1,
ou ses sels de métaux alcalins ou alcalino-terreux ou d'ammonium.

9. Dérivé de déhydroalanine selon la revendication 8, caractérisée par le fait que R désigne un reste méthyle ou éthyle.

10. Dérivé de déhydroalanine selon la revendication 8 ou 9, caractérisée par le fait que l'halogène est du chlore.

11. Dérivé de déhydroalanine selon l'une quelconque des revendications 8 à 10, caractérisée par le fait qu'elle est choisie parmi la N-ortho-toluyl déhydroalanine, la N-(2-méthoxy-5-chlorobenzoyl) déhydroalanine et la N-(2-méthoxybenzoyl)déhydroalanine.

12. Dérivé de déhydroalanine de formule: 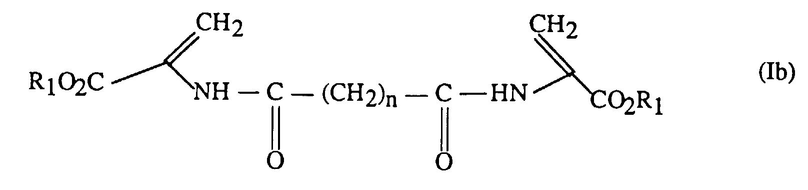 où n = 2, 3 ou 4,
R₁ = H ou alkyle en C₁-C₄ à chaîne linéaire ou ramifiée,
ou ses sels de métaux alcalins ou alcalino-terreux ou d'ammonium.

13. Dérivé de déhydroalanine selon la revendication 12, caractérisé par le fait que R₁ désigne un atome d'hydrogène et que n est égal à 2.

## Patentansprüche

1. Verwendung einer Dehydroalanin-Verbindung der Formel (I): 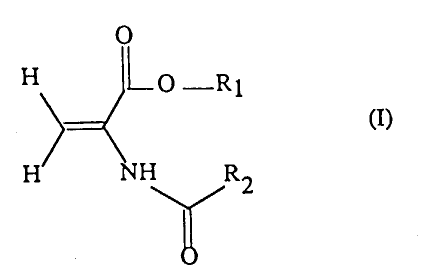 worin der Rest R₁ Wasserstoff oder einen linearen oder verzweigtren C₁₋₄-Alkylrest bedeutet, 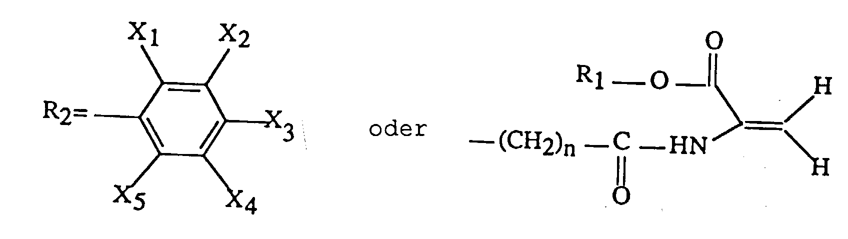 oder ein linearer oder verzweigter C₁₋₂₀-Alkylrest,
worin X₁, X₂, X₃, X₄ und X₅ = H, Halogen, ein Hydroxylrest, ein linearer oder verzweigter C₁₋₄-Alkyl- oder -Alkoxyrest, -OSO₃H, 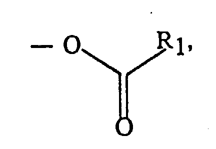 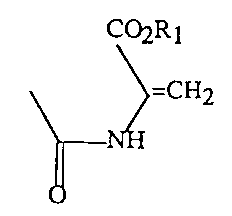 und n = 2,3 oder 4,
oder von deren Alkali- oder Erdalkali- oder Ammoniumsalzen
zur kosmetischen Behandlung der Haut, der Schleimhäute und/oder der Haare zum Schutz vor Auswirkungen durch oxidative Belastung.

2. Verwendung gemäß Anspruch 1 von Verbindungen der Formel (I), worin das Halogen Chlor ist.

3. Verwendung gemäß Anspruch 1 von Verbindungen der Formel (I), worin der C₁₋₄-Alkyl- oder -Alkoxyrest aus Methyl-, Ethyl-, Methoxy- und Ethoxyresten ausgewählt ist.

4. Kosmetische oder dermatologische Zusammensetzung, dadurch **gekennzeichnet**, daß sie mindestens eine in jedem der Ansprüche 1 bis 3 definierte Verbindung der Formel (I) in einem kosmetisch oder dermatologisch geeigneten Milieu enthält.

5. Zusammensetzugn gemäß Anspruch 4, dadurch gekennzeichnet, daß sie 0,001 bis 10 und vorzugsweise 0,01 bis 2 Gew.% mindestens einer in Anspruch 1 definierten Verbindung der Formel (I) enthält.

6. Zusammensetzung gemäß Anspruch 4 oder 5,
dadurch **gekennzeichnet**, daß sie mindestens eine Verbindung der Formel (I) enthält, ausgewählt aus N-Benzoyldehydroalanin, N-o-Toluyldehydroalanin, N- (2-Methoxy-5-chlorbenzoyl)dehydroalanin, N-(2-Methoxybenzoyl)dehydroalanin und aus dem von Bernsteinsäureamid abgeleiteten Dehydroalanin der Formel:

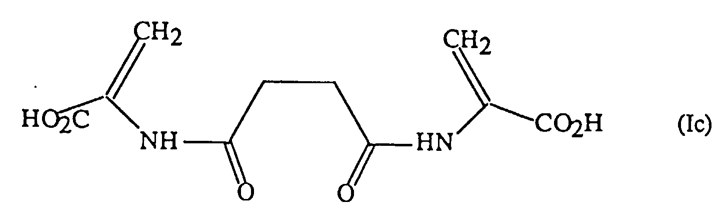

7. Kosmetische oder dermatologische Zusammensetzung gemäß einem der Ansprüche 4 bis 6,
dadurch **gekennzeichnet,** daß sie ausserdem mindestens ein kosmetisches oder dermatologisches Wirkstoffprinzip enthält, ausgewählt z.B. aus entzündungshemmenden Mitteln, Antiaknemitteln, Antipsoriasismitteln, Antipilzmitteln, antibakteriellen Mitteln, Mitteln gegen Haarausfall, Mitteln gegen die Schädigung des Haars, Filterstoffverbindungen, Vitaminen, keratolytischen Mitteln, hydratisierenden Mitteln, Mitteln gegen die Bildung freier Radikale, welche sich von denjenigen der Formel (I) unterscheiden, sowie aus Antioxidantien.

8. Dehydroalanin-Derivat der Formel: 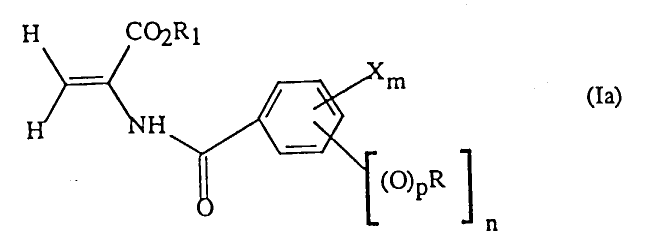
worin R₁ Wasserstoff bedeutet,
X ein Halogen bedeutet,
R einen linearen oder verzweigten C₁₋₄-Alkylrest bedeutet,
n + m = 1 bis 5, wobei m gleich Null sein kann,
p = 0 der 1,
oder deren Alkali- oder Erdalkali- oder Ammoniumsalze.

9. Dehydroalanin-Derivat gemäß Anspruch 8,
dadurch **gekennzeichnet**, daß
R einen Methyl- oder Ethylrest bedeutet.

10. Dehydroalanin-Derivat gemäß Anspruch 8 oder 9,
dadurch **gekennzeichnet,** daß
das Halogen Chlor ist.

11. Dehydroalanin-Derivat gemäß einem der Ansprüche 8 bis 10,
dadurch **gekennzeichnet,** daß
es aus N-o-Toluyldehydroalanin, N-(2-Methoxy-5-chlorbenzoyl)dehydroalanin und aus N-(2-Methoxybenzoyl)dehydroalanin ausgewählt ist.

12. Dehydroalanin-Derivat der Formel: 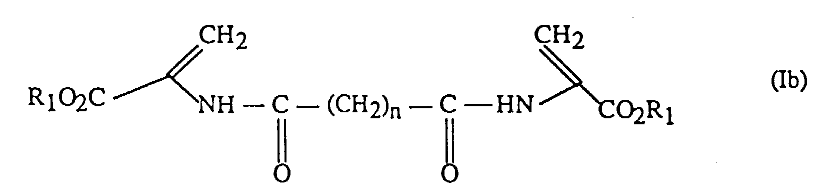
worin n = 2, 3 oder 4,
R₁ = H oder ein linearer oder verzweigter C₁₋₄-Alkylrest, oder deren Alkali- oder Erdalkali- oder Ammoniumsalze.

13. Dehydroalanin-Derivat gemäß Anspruch 12,
dadurch **gekennzeichnet,** daß R₁ ein Wasserstoffatom bedeutet und n gleich 2 ist.

## Claims

1. Use of a dehydroalanine of formula (I) 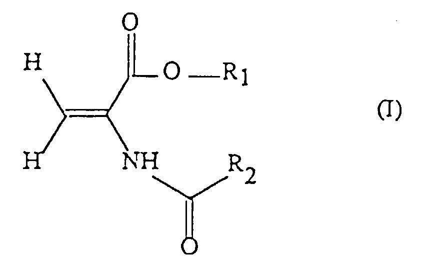 in which the R₁ radical denotes hydrogen or linear- or branched-chain C₁-C₄ alkyl, 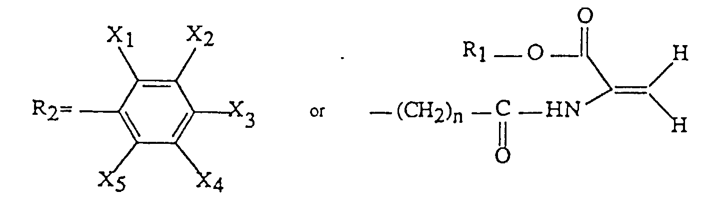 or linear- or branched-chain C₁-C₂₀ alkyl where X₁, X₂, X₃, X₄ and X₅ = H, halogen, hydroxyl, linear- or branched-chain C₁-C₄ alkyl or alkoxy, - OSO₃H, 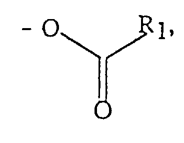 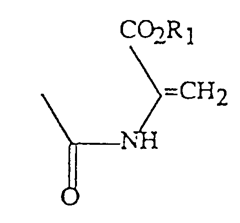 and n = 2, 3 or 4,
or of its alkali metal or alkaline-earth metal or ammonium salts in the cosmetic treatment of the skin, mucous membranes and/or hair for the purpose of protecting them against oxidative stress.

2. Use according to Claim 1 of compounds of formula (I) in which the halogen is chlorine.

3. Use according to Claim 1 of compounds of formula (I) in which the C₁-C₄ alkyl or alkoxy radical is chosen from the methyl, ethyl, methoxy and ethoxy radicals.

4. Cosmetic or dermatological composition, characterized in that it contains at least one compound of formula (I) as defined in any one of Claims 1 to 3 in a cosmetically or dermatologically acceptable medium.

5. Composition according to Claim 4, characterized in that it contains 0.001 weight % to 10 weight %, and preferably 0.01 weight % to 2 weight %, of at least one compound of formula (I) as defined in Claim 1.

6. Composition according to Claim 4 or 5, characterized in that it comprises at least one compound of formula (I) chosen from N-benzoyldehydroalanine, N-(ortho-toluyl)dehydroalanine, N-(2-methoxy-5-chlorobenzoyl)dehydroalanine, N-(2-methoxybenzoyl)dehydroalanine and the dehydroalanine derived from succinamide corresponding to the formula:

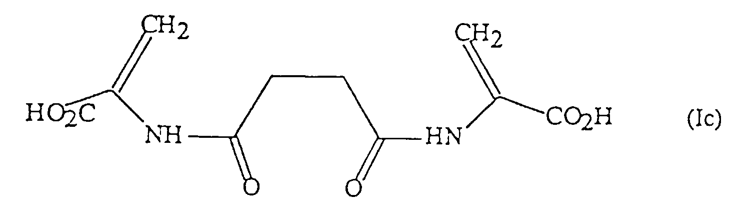

7. Cosmetic or dermatological composition according to any one of Claims 4 to 6, characterized in that it additionally contains at least one cosmetic or dermatological active principle chosen, for example, from antiinflammatories, antiacne agents, antipsoriatic agents, antifungal agents, antibacterial agents, agents for combating hair loss, antidandruff agents, screening agents, vitamins, keratolytic agents, humectants, agents for combating free radicals other than those of formula (I), and antioxidants.

8. Dehydroalanine derivative of formula: 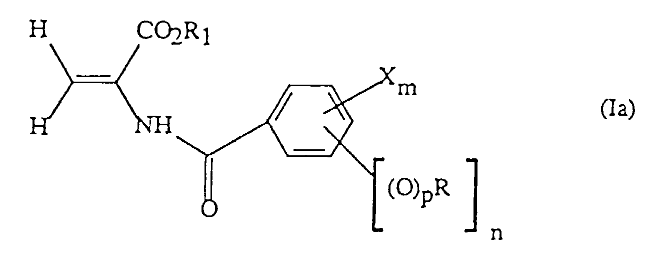 in which
R₁ denotes hydrogen,
X denotes a halogen,
R denotes a linear- or branched-chain C₁-C₄ alkyl residue,
n + m = 1 to 5, it being possible for m to be zero,
p = 0 or 1,
or its alkali metal or alkaline-earth metal or ammonium salts.

9. Dehydroalanine derivative according to Claim 8, characterized in that R denotes a methyl or ethyl residue.

10. Dehydroalanine derivative according to Claim 8 or 9, characterized in that the halogen is chlorine.

11. Dehydroalanine derivative according to any one of Claims 8 to 10, characterized in that it is chosen from N-(ortho-toluyl)dehydroalanine, N-(2-methoxy-5-chlorobenzoyl) dehydroalanine and N- (2-methoxybenzoyl) dehydroalanine.

12. Dehydroalanine derivative of formula: 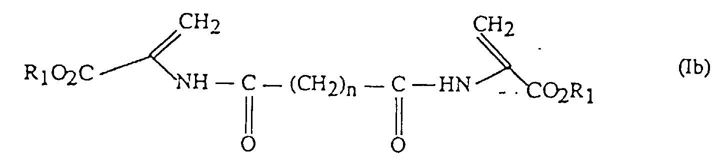 where
n = 2, 3 or 4,
R₁ = H or linear- or branched-chain C₁-C₄ alkyl,
or its alkali metal or alkaline-earth metal or ammonium salts.

13. Dehydroalanine derivative according to Claim 12, characterized in that R₁ denotes a hydrogen atom and in that n is equal to 2.
